# EUROPEAN PATENT SPECIFICATION

(11) **EP 1 496 115 B1**
(45) Date of publication and mention of the grant of the patent: **06.06.2012**
(21) Application number: 03746432.8
(22) Date of filing: 07.04.2003
(51) Int. Cl.: C12P 23/00

(54) **Process for obtaining canthaxanthin-producing microorganisms and for producing canthaxanthin**
Verfahren zur Gewinnung von Canthaxanthin-produzierenden Mikroorganismen und zur Herstellung von Canthaxanthin
Procédé pour l'obtention de microorganismes produisant de la canthaxanthine et pour production de la canthaxanthine

(30) Priority: 15.04.2002 JP 2002112240
(43) Date of publication of application: 12.01.2005
(73) Proprietor: JX Nippon Oil & Energy Corporation, Chiyoda-ku Tokyo 100-8162 (JP)
(72) Inventor: HIRASAWA, Kazuaki, c/o Nippon Oil & Energy Corporation, Tokyo 100-8162 (JP); TSUBOKURA, Akira, c/o Nippon Oil & Energy Corporation, Tokyo 100-8162 (JP); MIZUTA, Haruyoshi, c/o Nippon Oil & Energy Corporation, Tokyo 100-8162 (JP)
(74) Representative: Harding, Charles Thomas
(86) International application number: PCT/JP2003/004398
(87) International publication number: WO 2003/087358

(56) References cited:
- EP-A1- 0 769 551
- EP-A1- 1 138 208
- EP-A2- 0 872 554
- WO-A1-01/96591
- WO-A1-99/06586
- JP-A- 9 308 481
- TSUBOKURA A. ET AL.: 'Paracoccus carotinifaciens sp. nov., a new aerobic gram-negative astaxanthine-producing bacterium' INT. J. SYST. BACTERIOL. vol. 49, 1999, pages 277 - 282, XP002962170
- TRINIDAD D.M. ET AL.: 'Analysis of canthaxanthin and related pigments from Gordonia jacobaea mutants' J. AGRIC. FOOD CHEM. vol. 49, 2001, pages 1200 - 1202, XP002962180
- NELIS H.J. ET AL.: 'Reinvestigation of brevibacterium sp. strain KY-4313 as a source of canthaxanthin' APPLIED AND ENVIRONMENTAL MICROBIOLOGY vol. 55, no. 10, 1989, pages 2505 - 2510, XP002962181

## Description

### Technical Field

The present invention relates to a method for obtaining canthaxanthin-producing microorganisms, and a method for microbiologically producing canthaxanthin. Canthaxanthin, is useful as a natural red pigment for feed additives, food additives, etc.

### Background Art

As a method for improving the color tone of egg yolk, flesh, and skin of poultry such as chickens, addition of canthaxanthin to animal feeds are extensively employed in the world. Canthaxanthin is also used in the animal feed industry to improve the color tone of flesh or skin of seafood such as salmon, trout, red sea bream, or shrimp, and also used in the food industry as a coloring agent for foods and beverages.

It has been known that canthaxanthin is contained in certain species of mushrooms (Botanical Gazette, 112, 228-232, 1950), fish, and crustaceans. Examples of known canthaxanthin-producing microorganisms are those belonging to the genus *Brevibacterium* (Applied and Environmental Microbiology, 55 (10), 2505, 1989), those belonging to the genus *Rhodococcus* (JP Patent Publication (Unexamined Application) No. 2-138996), those belonging to the genus *Corynebacterium* (JP Patent Publication (Unexamined Application) No. 6-343482), and the bacterial strain E-396 belonging to a novel genus (JP Patent Publication (Unexamined Application) No. 2001-95500). As methods of chemical synthesis, oxidation of β-carotene (J. Amer. Chem. Soc., 78, 1427, 1956) and synthesis from 3-oxo-C15 phosphonium salt (Pure Appl. Chem., 51, 875, 1979) are known.

WO0196591 and EP1229126 disclose a process for the microbial production of carotenoid compounds wherein the ratio of carotenoid compounds formed is varied by controlling the concentration of oxygen dissolved in a liquid culture medium during the culture.

EP1138208 discloses a pigment-containing substance for feed additives consisting of a microorganisms culture precipitate which contains a high concentration of carotenoid compounds.

Miguel et al (2001, J Agric Food Chem vol 49, pages 1200-1202) disclose a collection of 43 mutant strains of *Gordonia jacobaea* obtained by means of ethyl methanesulfonate treatment. Canthaxanthin and other cartenoids were extracted from these mutants.

Nelis et al (1989, Applied and Environmental microbiology vol 55(10) pages 2505-2510) disclose the reevaluation of *Brevibacterium* sp strain KY-4313 for its potential to produce canthaxanthin.

WO9906586 discloses a *Paracoccus* species type strain DSM 11574 which produces and secretes carotenoids and a process for production of carotenoids.

### Disclosure of Invention

In the aforementioned method for chemically synthesizing canthaxanthin, however, an organic solvent is used. Thus, this method is problematic in terms of safety and the preferences for natural products these days. Conventional culture using microorganisms also has problems in that productivity is low and extraction from natural products is cost-intensive.

Regarding the E-396 strain that is known as a carotenoid compound-producing microorganism, its safety has been already assured and a method for producing highly concentrated carotenoid compounds comprising astaxanthin has been already reported. However, the ratio of canthaxanthin to total carotenoid produced is low.

As an alternative to canthaxanthin, capsanthin that is extracted from a paprika plant may be used to improve the color tone of chicken egg yolk. Since capsanthin is very unstable in terms of heat, light, or the like, and growth of paprika is significantly influenced by weather, it is difficult to provide capsanthin at industrially stable levels.

Accordingly, a method for producing canthaxanthin, which is highly safe and stably provided at a low price, is desired.

As a result of intensive and extensive studies aimed at the solution of the aforementioned problems, the present inventors have found that microorganisms in which the ratio of canthaxanthin is high relative to the total amount of carotenoid produced could be easily obtained by mutating astaxanthin-producing microorganisms. This led to the completion of the present invention.

Specifically, the present invention provides the following means.
1. A method for obtaining canthaxanthin-producing microorganisms comprising the steps of:
   (i) inducing mutation in astaxanthin-producing microorganisms having a ratio about 2 to 20% of canthaxanthin produced by mass relative to the total amount of carotenoid produced in which the nucleotide sequence of DNA corresponding to its 16S ribosomal RNA is 98% or higher homologous to the nucleotide sequence as shown in SEQ ID NO: 1; and
   (ii) selecting a mutant having a ratio of at least 60% of canthaxanthin produced by mass relative to the total amount of carotenoid produced.
2. The method according to paragraph 1 above, wherein each of the ratios of β-cryptoxanthin, zeaxanthin, 3-hydroxychinenone, asteroidenone, adonirubin, adonixanthin, and astaxanthin produced from the canthaxanthin-producing microorganisms is less than 20% by mass relative to the total amount of carotenoid produced.
3. The method according to paragraphs 1 or 2 above, wherein the astaxanthin-producing microorganisms are selected from the E-396 strain (FERM BP-4283) and a mutant thereof, and the A-581-1 strain (FERM BP-4671) and a mutant thereof.
4. A method for microbiologically producing canthaxanthin comprising culturing the canthaxanthin-producing microorganisms obtained by a method according to any one of paragraphs 1 to 3 above, and recovering canthaxanthin produced.

Also described herein are
(1) A method for producing canthaxanthin comprising steps of: inducing mutation in astaxanthin-producing microorganisms in which the nucleotide sequence of DNA corresponding to its 16S ribosomal RNA is substantially homologous to the nucleotide sequence as shown in SEQ ID NO: 1; obtaining canthaxanthin-producing microorganisms by selecting a mutant having a higher ratio of canthaxanthin produced (% by mass) relative to the amount of carotenoid produced than that of a parent strain; and recovering canthaxanthin or a carotenoid mixture comprising canthaxanthin from the culture product of the canthaxanthin-producing microorganisms.
(2) The method according to (1) above, wherein the ratio of canthaxanthin produced from the canthaxanthin-producing microorganisms is at least 40% by mass relative to the total amount of carotenoid produced.
(3) The method according to (1) above, wherein each of the ratios of β-cryptoxanthin, zeaxanthin, 3-hydroxyechinenone, asteroidenone, adonirubin, adonixanthin, and astaxanthin produced from the canthaxanthin-producing microorganisms is less than 20% by mass relative to the total amount of carotenoid produced.
(4) The method according to any one of (1) to (3) above, wherein the astaxanthin-producing microorganisms are selected from the E-396 strain (which has been deposited at the National Institute of Bioscience and Human-Technology (the International Patent Organism Depositary of the National Institute of Advanced Industrial Science and Technology; AIST Tsukuba Central 6, 1-1-1 Higashi, Tsukuba, Ibaraki, Japan) as of April 27, 1993, under the accession number of FERM BP-4283) and a mutant thereof, and the A-581-1 strain (which has been deposited at the National Institute of Bioscience and Human-Technology (the International Patent Organism Depositary of the National Institute of Advanced Industrial Science and Technology; AIST Tsukuba Central 6, 1-1-1 Higashi, Tsukuba, Ibaraki, Japan) as of May 20, 1994, under the accession number of FERM BP-4671) and a mutant thereof.

The present invention is hereafter described in more detail.

In the method according to the present invention, an astaxanthin-producing microorganism is used as a parent strain for mutation. An example of such a microorganism is an astaxanthin-producing microorganism in which the nucleotide sequence of DNA corresponding to its 16S ribosomal RNA is substantially homologous to the nucleotide sequence as shown in SEQ ID NO: 1. The term "substantially homologous" used herein refers to homology of 98% or higher in view of, for example, error frequency in nucleotide sequencing of DNA.

Specific examples of astaxanthin-producing microorganisms having a sequence substantially homologous to the above sequence include the E-396 strain (FERM BP-4283), the A-581-1 strain (FERM BP-4671), various mutants obtained by mutating and improving the E-396 or A-581-1 strain, and related species thereof. The nucleotide sequence of DNA as shown in SEQ ID NO: 1 corresponds to ribosomal RNA of the E-396 strain, and the nucleotide sequence of DNA as shown in SEQ ID NO: 2 corresponds to ribosomal RNA of the A-581-1 strain. Homology of nucleotide sequences of 16S ribosomal RNA between the E-396 strain and the A-581-1 strain is 99.4%, and this indicates that they are closely related strains. Accordingly, these strains form a group of carotenoid-producing bacteria. In the method according to the present invention, a parent strain used in mutation is defined as an astaxanthin-producing microorganism in which the nucleotide sequence of DNA corresponding to its 16S ribosomal RNA is 98% or more homologous to the nucleotide sequence as shown in SEQ ID NO: 1, i.e., the E-396 strain, the A-581-1 strain, mutants of the E-396 or the A-581-1 strain, and related species thereof.

The E-396 strain, which is exemplified as the astaxanthin-producing microorganism used in the present invention, is described. This strain was newly isolated by the present inventors and deposited at the National Institute of Bioscience and Human-Technology (the International Patent Organism Depositary of the National Institute of Advanced Industrial Science and Technology) as of April 27, 1993, under the accession number of FERM BP-4283. A more specific example of another microorganism is the A-581-1 strain. This strain was newly isolated by the present inventors and deposited at the National Institute of Bioscience and Human-Technology (the International Patent Organism Depositary of the National Institute of Advanced Industrial Science and Technology) as of May 20, 1994, under the accession number of FERM BP-4671.

In the present invention, the method for mutating astaxanthin-producing microorganisms is not particularly limited as long as the method can induce mutation. Examples of methods which can be used include: chemical methods using mutating agents such as N-methyl-N'-nitro-N-nitrosoguanidine (NTG) or ethyl methanesulfonate (EMS); physical methods such as ultraviolet irradiation or X-ray irradiation; and biological methods using gene recombination or transposon. This mutation may be carried out once. Alternatively, mutation may be carried out twice or more by, for example, preparing a mutant of the astaxanthin-producing microorganism by this mutation and further mutating the resulting mutant.

In the present invention, a mutant in which the ratio of canthaxanthin produced is particularly high relative to the total amount of carotenoid can be selected from among mutants obtained by mutating astaxanthin-producing microorganisms by analyzing the carotenoid compound in the culture solution of the mutant.

For example, this culture method is carried out in the following manner. Specifically, culture is conducted in a medium containing a component that is necessary for the growth of the canthaxanthin-producing microorganisms and that generates a carotenoid compound. Culture method may be shake culture using a test tube, flask, or the like, or via aeration agitation culture. A carotenoid compound may be analyzed by any method as long as the carotenoid compound can be separated and detected thereby. For example, high-performance liquid chromatography, thin-layer chromatography, or paper chromatography can be employed.

In the present invention, canthaxanthin-producing microorganisms are obtained by selecting a mutant in which the ratio of canthaxanthin is at least 60% by mass relative to the total amount of carotenoid. The term "total amount of carotenoid" used herein refers to the total amount of carotenoid compounds such as astaxanthin, canthaxanthin, adonixanthin, β-carotene, echinenone, zeaxanthin, β-cryptoxanthin, 3-hydroxyechinenone, asteroidenone, or adonirubin.

The astaxanthin-producing microorganisms such as the E-396 strain simultaneously produce various carotenoid compounds such as astaxanthin, canthaxanthin, adonixanthin, β-carotene, echinenone, zeaxanthin, β-cryptoxanthin, 3-hydroxyechinenone, asteroidenone, or adonirubin. Thus, the ratio of canthaxanthin relative to the total amount of carotenoid is low, and it is about 2% to 20%.

A mutant that is selected induces mutation in astaxanthin-producing microorganisms and the ratio of canthaxanthin produced is particularly high relative to the total amount of carotenoid. The minimum condition for the ratio of canthaxanthin employed as a standard for selection is that this ratio is higher than that of the parent strain before mutation for producing canthaxanthin. Also described herein is a mutant selected that produces carotenoid containing preferably at least 40% canthaxanthin by mass, and more preferably at least 60% by mass, based on the total amount of carotenoid produced.

The biosynthesis of astaxanthin is deduced as follows. The 6-membered rings at both terminuses of β-carotene are modified by ketolase and hydroxylase. If the deletion of this hydroxylase is complete, it is deduced that only β-carotene, echinenone, and canthaxanthin are produced, and that β-cryptoxanthin, zeaxanthin, 3-hydroxyechinenone, asteroidenone, adonirubin, adonixanthin, and astaxanthin, which are in need of hydroxylase, are not produced. If the deletion of this hydroxylase is incomplete, it is deduced that the ratios of β-cryptoxanthin, zeaxanthin, 3-hydroxyechinenone, asteroidenone, adonirubin, adonixanthin, and astaxanthin relative to the total amount of carotenoid are lowered. Accordingly, as another effective means to select canthaxanthin-producing microorganisms from among mutants, selection can be made based on the phenomenon that the ratios of β-cryptoxanthin, zeaxanthin, 3-hydroxyechinenone, asteroidenone, adonirubin, adonixanthin, and astaxanthin relative to the total amount of carotenoid are low. Selection can be made based on the phenomenon that the ratio of each compound relative to the total carotenoid is preferably less than 20% by mass, and more preferably less than 10% by mass.

In the present invention, the method for culturing canthaxanthin-producing microorganisms to recover canthaxanthin or a carotenoid mixture comprising canthaxanthin may be any method as long as canthaxanthin is produced. Examples of methods that can be used are as follows. Specifically, a medium comprises carbon sources, nitrogen sources, inorganic salts, and if necessary particular nutritional requirements (e.g., vitamins, amino acids, or nucleic acids) that are necessary in the growth of canthaxanthin-producing microorganisms. Examples of carbon sources include: saccharides such as glucose, sucrose, fructose, trehalose, mannose, mannitol, and maltose; organic acids such as acetic acid, fumaric acid, citric acid, propionic acid, malic acid, and malonic acid; and alcohols such as ethanol, propanol, butanol, pentanol, hexanol, and isobutanol. The amount of carbon sources added varies depending their types, and it is generally 1 to 100 g, preferably 2 to 50 g per liter of medium. Examples of nitrogen sources include potassium nitrate, ammonium nitrate, ammonium chloride, ammonium sulfate, ammonium phosphate, ammonia, and urea. These may be used alone or in combination of two or more. The amount of nitrogen sources added varies depending on their types, and it is generally 0.1 to 20 g, preferably 1 to 10 g per liter of medium. Examples of inorganic salts include monobasic potassium phosphate, dibasic potassium phosphate, disodium hydrogen-phosphate, magnesium sulfate, magnesium chloride, iron sulfate, iron chloride, manganese sulfate, manganese chloride, zinc sulfate, zinc chloride, copper sulfate, calcium chloride, calcium carbonate, and sodium carbonate. These may be used alone or in combination of two or more. The amount of inorganic salts added varies depending on their types, and it is generally 0.1 mg to 10 g per liter of medium. Examples of particular nutritional requirements include vitamins, nucleic acids, yeast extract, peptone, meat extract, malt extract, corn steep liquor, dry yeast, soybean cake, soybean oil, olive oil, corn oil, and linseed oil. These may be used alone or in combination of two or more. The amounts of particular nutritional requirements added vary depending on their types, and it is generally 0.01 mg to 100 g per liter of medium. The pH of the medium is adjusted to between 2 and 12, preferably between 6 and 9. Shake culture or aeration agitation culture is carried out at 10°C to 70°C, preferably 20°C to 35°C generally for 1 to 20 days, and preferably for 2 to 9 days.

Subsequently, moisture is removed from the thus obtained culture solution. The amount of moisture, which should be removed from the culture solution in order to obtain a canthaxanthin-containing substance, varies depending on conditions such as a pigment content of the culture solution. In general, filtration is first carried out, and a precipitate is dehydrated if moisture should be further removed. Filtration can be carried out by commonly employed methods such as filtration or centrifugation. When the content of carotenoid compound in the precipitate should be increased, moisture can be removed by dehydrating the precipitate. Examples of dehydration methods include general spray drying, drum-drying, and freeze-drying.

The thus obtained culture precipitate of canthaxanthin-containing microorganisms can be used in that state as a substance containing a pigment for feed additives. When the canthaxanthin and the carotenoid compound obtained by the method of the present invention are used as a coloring agent for feeds, etc., antioxidants such as butylhydroxytoluene, ethoxyquin, or vitamin E can be added to prevent the canthaxanthin and the carotenoid compound from being degraded. Further, the surfaces thereof may be covered with gelatin, etc.

### Best Mode for Carrying Out the Invention

The present invention is hereafter described in more detail with reference to the examples but is not limited thereto.

### [Example 1]

The E-396 strain (FERM BP-4283, the ratio of canthaxanthin produced: 7.4% by mass) was allowed to stand at 28°C for 30 minutes and subjected to mutation with 150 mg/L NTG (N-methyl-N'-nitro-N-nitrosoguanidine). A medium (6 ml) having a composition as shown in Table 1 was placed in a test tube (inner diameter: 18 mm) and steam-sterilized at 121°C for 15 minutes to prepare a test tube medium. Each of the 400 mutants that were subjected to colony isolation was inoculated on a test tube medium by means of an inoculating loop, and reciprocal shake culture (300 rpm) was carried out at 28°C for 4 days. Subsequently, this culture product was centrifuged, and the resultant cells were analyzed for their carotenoid compound by high-performance liquid chromatography. As a result, one strain that exhibited a ratio of canthaxanthin relative to the total amount of carotenoid produced of 60% by mass or higher was obtained. The results of analysis of the carotenoid compound in this strain are shown in Table 2.

**Table 1**

| Composition | Amount added, g/L |
|---|---|
| Yeast extract | 20 |
| Peptone | 5 |
| Sucrose | 50 |
| KH₂PO₄ | 1.5 |
| Na₂HPO₄·12H₂O | 3.8 |
| MgSO₄·7H₂O | 0.5 |
| FeSO₄·7H₂O | 0.01 |
| CaCl₂·2H₂O | 0.01 |
| Na₂CO₃ | The amount with which the pH of the medium reaches 7 |

**Table 2**

| Carotenoid compound | Concentration of product per culture solution mg/L | Ratio of product % by mass |
|---|---|---|
| β-Carotene | 0.3 | 7.1 |
| Echinenone | 0.5 | 11.9 |
| 3-Hydroxyechinenone | 0.0 | 0.0 |
| Canthaxanthin | 2.7 | 64.3 |
| Adonirubin | 0.6 | 14.3 |
| β-Cryptoxanthin | 0.0 | 0.0 |
| Astaxanthin | 0.1 | 2.4 |
| Asteroidenone | 0.0 | 0.0 |
| Adonixanthin | 0.0 | 0.0 |
| Zeaxanthin | 0.0 | 0.0 |

### [Example 2]

The E-396 strain (FERM BP-4283) was mutated with NTG, colonies with a deep red color were selected, and variant Y-1071 (the ratio of canthaxanthin produced: 6.5% by mass) with enhanced productivity of astaxanthin was obtained. This Y-1071 strain was further mutated with NTG. A medium (6 ml) having a composition as shown in Table 1 was placed in a test tube (inner diameter: 18 mm) and steam-sterilized at 121°C for 15 minutes to prepare a test tube medium. Each of the 1,000 mutants that were subjected to colony isolation was inoculated on a test tube medium by means of an inoculating loop, and reciprocal shake culture (300 rpm) was carried out at 28°C for 4 days. Subsequently, this culture product was centrifuged, and the resultant cells were analyzed for their carotenoid compound by high-performance liquid chromatography. As a result, two strains in which each of the ratios of β-cryptoxanthin, zeaxanthin, 3-hydroxyechinenone, asteroidenone, adonirubin, adonixanthin, and astaxanthin relative to the total amount of carotenoid was less than 10% by mass were obtained. The results of analysis of the carotenoid compound in these two strains are shown in Table 3 and Table 4.

**Table 3**

| Carotenoid compound | Concentration of product per culture solution mg/L | Ratio of product % by mass |
|---|---|---|
| β-Carotene | 0.8 | 8.1 |
| Echinenone | 1.1 | 11.1 |
| 3-Hydroxyechinenone | 0.0 | 0.0 |
| Canthaxanthin | 7.0 | 70.7 |
| Adonirubin | 0.9 | 9.1 |
| β-Cryptoxanthin | 0.0 | 0.0 |
| Astaxanthin | 0.1 | 1.0 |
| Asteroidenone | 0.0 | 0.0 |
| Adonixanthin | 0.0 | 0.0 |
| Zeaxanthin | 0.0 | 0.0 |

**Table 4**

| Carotenoid compound | Concentration of product per culture solution mg/L | Ratio of product % by mass |
|---|---|---|
| β-Carotene | 0.6 | 5.5 |
| Echinenone | 0.7 | 6.4 |
| 3-Hydroxyechinenone | 0.0 | 0.0 |
| Canthaxanthin | 9.6 | 88.1 |
| Adonirubin | 0.0 | 0.0 |
| β-Cryptoxanthin | 0.0 | 0.0 |
| Astaxanthin | 0.0 | 0.0 |
| Asteroidenone | 0.0 | 0.0 |
| Adonixanthin | 0.0 | 0.0 |
| Zeaxanthin | 0.0 | 0.0 |

### [Example 3]

The A-581-1 strain (FERM BP-4671, the ratio of canthaxanthin produced: 5.3% by mass) was mutated by irradiating it with ultraviolet light using a UV lamp. A medium (6 ml) having a composition as shown in Table 1 was placed in a test tube (inner diameter: 18 mm) and steam-sterilized at 121°C for 15 minutes to prepare a test tube medium. Each of the 300 mutants that were subjected to colony isolation was inoculated on a test tube medium by means of an inoculating loop, and reciprocal shake culture (300 rpm) was carried out at 28°C for 4 days. Subsequently, this culture product was centrifuged, and the resultant cells were analyzed for their carotenoid compound by high-performance liquid chromatography. As a result, one strain that exhibited a ratio of canthaxanthin at 60% by mass or higher relative to the total amount of carotenoid was obtained. The results of analysis of the carotenoid compound in this strain are shown in Table 5.

**Table 5**

| Carotenoid compound | Concentration of product per culture solution mg/L | Ratio of product % by mass |
|---|---|---|
| β-Carotene | 0.2 | 8.7 |
| Echinenone | 0.3 | 13.0 |
| 3-Hydroxyechinenone | 0.0 | 0.0 |
| Canthaxanthin | 1.5 | 65.2 |
| Adonirubin | 0.2 | 8.7 |
| β-Cryptoxanthin | 0.0 | 0.0 |
| Astaxanthin | 0.1 | 4.3 |
| Asteroidenone | 0.0 | 0.0 |
| Adonixanthin | 0.0 | 0.0 |
| Zeaxanthin | 0.0 | 0.0 |

### Industrial Applicability

The present invention provides a method for producing canthaxanthin, which is inexpensive, stably provided, and highly safe.

## Claims

1. A method for obtaining canthaxanthin-producing microorganisms comprising the steps of:
(i) inducing mutation in astaxanthin-producing microorganisms having a ratio about 2 to 20% of canthaxanthin produced by mass relative to the total amount of carotenoid produced in which the nucleotide sequence of DNA corresponding to its 16S ribosomal RNA is 98% or higher homologous to the nucleotide sequence as shown in SEQ ID NO: 1; and
(ii) selecting a mutant having a ratio of at least 60% of canthaxanthin produced by mass relative to the total amount of carotenoid produced.

2. The method according to claim 1, wherein each of the ratios of cryptoxanthin, zeaxanthin, 3-hydroxychinenone, asteroidenone, adonirubin, adonixanthin, and astaxanthin produced from the canthaxanthin-producing microorganisms is less than 20% by mass relative to the total amount of carotenoid produced.

3. The method according to claim 1 or 2, wherein the astaxanthin-producing microorganisms are selected from the E-396 strain (FERM BP-4283) and a mutant thereof, and the A-581-1 strain (FERM BP-4671) and a mutant thereof.

4. A method for microbiologically producing canthaxanthin comprising culturing the canthaxanthin-producing microorganisms obtained by a method according to any one of claims 1 to 3, and recovering canthaxanthin produced.

## Patentansprüche

1. Verfahren zum Erhalten Canthaxanthin produzierender Mikroorganismen, das die folgenden Stufen umfasst:
(i) Induzieren einer Mutation in Astaxanthin produzierenden Mikroorganismen, bei denen ein Verhältnis von 2 bis 20 Masse-% produziertes Canthaxanthin im Vergleich zu der gesamten Menge an produziertem Carotinoid vorliegt, bei welchen die Nukleotidsequenz von DNA, die ihrer 16S ribosomalen RNA entspricht, 98 % oder mehr homolog zu der in SEQ ID NO: 1 gezeigten Nukleotidsequenz ist, und
(ii) Selektieren einer Mutante mit einem Verhältnis von wenigstens 60 Masse-% produziertem Canthaxanthin im Verhältnis zu der Gesamtmenge an hergestelltem Carotinoid.

2. Verfahren gemäß Anspruch 1, wobei jedes der Verhältnisse von produziertem β-Cryptoxanthin, Zeaxanthin, 3-Hydroxychinenon, Asteroidenon, Adonirubin, Adonixanthin und Astaxanthin der Canthaxanthin produzierenden Mikroorganismen weniger als 20 Masse-% relativ zu der Gesamtmenge an produziertem Carotinoid beträgt.

3. Verfahren gemäß Anspruch 1 oder 2, wobei die Astaxanthin produzierenden Mikroorganismen ausgewählt sind aus dem Stamm E-396 (FERM BP-4283) und einer Mutante davon und dem Stamm A-581-1 (FERM BP-4671) und einer Mutante davon.

4. Verfahren zur mikrobiologischen Produktion von Canthaxanthin, das das Kultivieren der durch ein Verfahren gemäß einem der Ansprüche 1 bis 3 erhaltenen Canthaxanthin produzierenden Mikroorganismen und Gewinnen von produziertem Canthaxanthin umfasst.

## Revendications

1. Procédé d'obtention de micro-organismes produisant de la canthaxanthine comprenant les étapes consistant à :
(i) induire une mutation dans des micro-organismes produisant de l'astaxanthine présentant un rapport d'environ 2 à 20 % de canthaxanthine produite en masse par rapport à la quantité totale de caroténoïde produite, où la séquence nucléotidique de l'ADN correspondant à son ARN ribosomique 16S est homologue à 98 % ou plus avec la séquence nucléotidique telle que représentée par SEQ ID NO : 1 ; et
(ii) sélectionner un mutant présentant un rapport d'au moins 60 % de canthaxanthine produite en masse par rapport à la quantité totale de caroténoïde produite.

2. Procédé selon la revendication 1, dans lequel chacun des rapports des β-cryptoxanthine, zéaxanthine, 3-hydroxychinénone, astéroïdénone, adonirubine, adonixanthine et astaxanthine produites à partir des micro-organismes est inférieur à 20 % en masse par rapport à la quantité totale de caroténoïde produite.

3. Procédé selon la revendication 1 ou 2, dans lequel les micro-organismes produisant de l'astaxanthine sont choisis parmi la souche E-396 (FERM BP-4283) et un mutant de celle-ci et la souche A-581-1 (FERM BP-4671) et un mutant de celle-ci.

4. Procédé de production de manière microbiologiqae de la canthaxanthine comprenant la culture des micro-organismes produisant de la canthaxanthine obtenus par un procédé selon l'une quelconque des revendications 1 à 3, et la récupération de la canthaxanthine produite.


### SEQUENCE LISTING

<110> Nippon Oil Corporation
<120> Method for producing canthaxanthin
<130> PH-1759CL
<140>
   <141>
<160> 2
   <170> PatentIn Ver. 2.0
<210> 1
   <211> 1452
   <212> DNA
   <213> Unknown
<220>
   <223> Description of Unknown Organism:E-396
<400> 1
   agtttgatcc tggctcagaa cgaacgctgg cggcaggctt aacacatgca agtcgagcga 60
   gaccttcggg tctagcggcg gacgggtgag taacgcgtgg gaacgtgccc ttctctacgg 120
   aatagccccg ggaaactggg agtaataccg tatacgccct ttgggggaaa gatttatcgg 180
   agaaggatcg gcccgcgttg gattaggtag ttggtggggt aatggcccac caagccgacg 240
   atccatagct ggtttgagag gatgatcagc cacactggga ctgagacacg gcccagactc 300
   ctacgggagg cagcagtggg gaatcttaga caatgggggc aaccctgatc tagccatgcc 360
   gcgtgagtga tgaaggcctt agggttgtaa agctctttca gctgggaaga taatgacggt 420
   accagcagaa gaagccccgg ctaactccgt gccagcagcc gcggtaatac ggagggggct 480
   agcgttgttc ggaattactg ggcgtaaagc gcacgtaggc ggactggaaa gtcagaggtg 540
   aaatcccagg gctcaacctt ggaactgcct ttgaaactat cagtctggag ttcgagagag 600
   gtgagtggaa ttccgagtgt agaggtgaaa ttcgtagata ttcggaggaa caccagtggc 660
   gaaggcggct cactggctcg atactgacgc tgaggtgcga aagcgtgggg agcaaacagg 720
   attagatacc ctggtagtcc acgccgtaaa cgatgaatgc cagacgtcgg caagcatgct 780
   tgtcggtgtc acacctaacg gattaagcat tccgcctggg gagtacggtc gcaagattaa 840
   aactcaaagg aattgacggg ggcccgcaca agcggtggag catgtggttt aattcgaagc 900
   aacgcgcaga accttaccaa cccttgacat ggcaggaccg ctggagagat tcagctttct 960
   cgtaagagac ctgcacacag gtgctgcatg gctgtcgtca gctcgtgtcg tgagatgttc 1020
   ggttaagtcc ggcaacgagc gcaacccacg tccctagttg ccagcaattc agttgggaac 1080
   tctatggaaa ctgccgatga taagtcggag gaaggtgtgg atgacgtcaa gtcctcatgg 1140
   gccttacggg ttgggctaca cacgtgctac aatggtggtg acagtgggtt aatccccaaa 1200
   agccatctca gttcggattg tcctctgcaa ctcgagggca tgaagttgga atcgctagta 1260
   atcgcggaac agcatgccgc ggtgaatacg ttcccgggcc ttgtacacac cgcccgtcac 1320
   accatgggag ttggttctac ccgacgacgn tgcgctaacc ttcggggggc aggcggccac 1380
   ggtaggatca gcgactgggg tgaagtcgta acaaggtagc cgtaggggaa cctgcggctg 1440
   gatcacctcc tt 1452
<210> 2
   <211> 1426
   <212> DNA
   <213> Unknown
<220>
   <223> Description of Unknown Organism:A-581-1
<400> 2
   tagagtttga tcctggctca gaacgaacgc tggcggcagg cttaacacat gcaagtcgag 60
   cgagaccttc gggtctagcg gcggacgggt gagtaacgcg tgggaacgtg cccttctcta 120
   cggaatagcc ccgggaaact gggagtaata ccgtatacgc cctttggggg aaagatttat 180
   cggagaagga tcggcccgcg ttggattagg tagttggtga ggtaacggct caccaagccg 240
   acgatccata gctggtttga gaggatgatc agccacactg ggactgagac acggcccaga 300
   ctcctacggg aggcagcagt ggggaatctt agacaatggg ggcaaccctg atctagccat 360
   gccgcgtgag tgatgaaggc cttagggttg taaagctctt tcagctggga agataatgac 420
   ggtaccagca gaagaagccc cggctaactc cgtgccagca gccgcggtaa tacggagggg 480
   gctagcgttg ttcggaatta ctgggcgtaa agcgcacgta ggcggactgg aaagtcagag 540
   gtgaaatccc agggctcaac cttggaactg cctttgaaac tatcagtctg gagttcgaga 600
   gaggtgagtg gaattccgag tgtagaggtg aaattcgtag atattcggag gaacaccagt 660
   ggcgaaggcg gctcactggc tcgatactga cgctgaggtg cgaaagcgtg gggagcaaac 720
   aggattagat accctggtag tccacgccgt aaacgatgaa tgccagacgt cggcaagcat 780
   gcttgtcggt gtcacaccta acggattaag cattccgcct ggggagtacg gtcgcaagat 840
   taaaactcaa aggaattgac gggggcccgc acaagcggtg gagcatgtgg tttaattcga 900
   agcaacgcgc agaaccttac caacccttga catggcagga ccgctggaga gattcagctt 960
   tctcgtaaga gacctgcaca caggtgctgc atggctgtcg tcagctcgtg tcgtgagatg 1020
   ttcggttaag tccggcaacg agcgcaaccc acgtccctag ttgccagcat tcagttgggc 1080
   actctatgga aactgccggt gataagccgg aggaaggtgt ggatgacgtc aagtcctcat 1140
   ggcccttacg ggttgggcta cacacgtgct acaatggtgg tgacagtggg ttaatcccca 1200
   aaagccatct cagttcggat tgtcctctgc aactcgaggg catgaagttg gaatcgctag 1260
   taatcgcgga acagcatgcc gcggtgaata cgttcccggg ccttgtacac accgcccgtc 1320
   acaccatggg agttggttct acccgacgac gctgcgctaa cccttcgggg aggcaggcgg 1380
   ccacggtagg atcagcgact ggggtgaagt cgtaacaagg tagcca                1426